# EUROPÄISCHE PATENTANMELDUNG

(11) **EP 4 620 399 A1**
(43) Veröffentlichungstag der Anmeldung: **24.09.2025**
(21) Anmeldenummer: 24192603.9
(22) Anmeldetag: 02.08.2024
(51) Int. Cl.: A61B 6/03, A61B 6/10, A61B 6/04

(54) **COMPUTERTOMOGRAPHIEGERÄT MIT EINER STRAHLENSCHUTZVORRICHTUNG ZUR ABDECKUNG EINER ÖFFNUNG**

(71) Anmelder: Siemens Healthineers AG, 91301 Forchheim (DE)
(72) Erfinder: RAMSAUER, Martin, 90602 Pyrbaum (DE); LÖWEN, Benjamin, 73527 Schwäbisch Gmünd (DE); DEDERICHS, David, 91099 Poxdorf (DE); FEHRE, Jens, 91353 Hausen (DE); KRIEGER, Daniel, 96178 Pommersfelden (DE)
(74) Vertreter: Siemens Healthineers Patent Attorneys

(57) **Zusammenfassung**

Die Erfindung betrifft ein Computertomographiegerät, aufweisend eine Gantry mit einer Öffnung und eine Strahlenschutzvorrichtung zur Abdeckung der Öffnung,
- wobei die Strahlenschutzvorrichtung eine Haltestruktur, einen Strahlenschutzvorhang und eine Schwenkvorrichtung aufweist,
- wobei ein Untersuchungsobjekt entlang einer Systemachse der Gantry in die Öffnung einführbar ist, wenn sich die Haltestruktur in einer ersten Position relativ zu der Gantry befindet,
- wobei die Haltestruktur mittels der Schwenkvorrichtung derart relativ zu der Gantry um eine Schwenkachse schwenkbar gelagert ist, dass durch eine erste Schwenkbewegung der Haltestruktur um die Schwenkachse die Haltestruktur relativ zu der Gantry von der ersten Position bis zu einer zweiten Position abgesenkt werden kann,
- wobei der Strahlenschutzvorhang an der Haltestruktur angeordnet ist, insbesondere an der Haltestruktur befestigt ist,
- wobei ein unterer Bereich des Strahlenschutzvorhangs von der Haltestruktur herabhängt.

## Beschreibung

Die Erfindung betrifft ein Computertomographiegerät (CT-Gerät).

Mobile Computertomographiegeräte werden immer mehr in Umgebungen eingesetzt, die ursprünglich nicht für den Betrieb eines Geräts, das ionisierende Streustrahlung aussendet, eingerichtet waren. Beispiele für solche Umgebungen sind Intensivstationen, Operations- und Interventionsräume und Mobile Stroke Units. Um die Umgebung des Computertomographiegeräts vor Streustrahlung zu schützen, können Verkleidungsteile des Computertomographiegeräts Schichten aus einem Strahlenschutzmaterial, beispielsweise aus Blei, aufweisen. Ferner sind Maßnahmen erforderlich, um die Umgebung des Computertomographiegeräts vor einer Streustrahlung, die aus einer Öffnung des Computertomographiegeräts, beispielsweise an einer Vorderseite oder an einer Rückseite einer Gantry des Computertomographiegeräts, austritt, zu schützen. Diese Maßnahmen zum Strahlenschutz sollten möglichst flexibel an einen Platzbedarf für Körperteile und/oder Zubehörteile, die mit dem Untersuchungsobjekt verbunden sind, angepasst werden können.

Als Stand der Technik sind hierbei die US 8 057 097 B1, US 2021 / 0 369 214 A1 und US 2023 / 0 094 920 A1 zu nennen.

Die Erfindung hat die Aufgabe, einen verbesserten Schutz einer Umgebung eines Computertomographiegeräts vor einer Streustrahlung, die aus einer Öffnung einer Gantry des Computertomographiegeräts austritt, zu ermöglichen. Jeder Gegenstand eines unabhängigen Anspruchs löst diese Aufgabe. In den abhängigen Ansprüchen sind weitere vorteilhafte Aspekte der Erfindung berücksichtigt. Unabhängig vom grammatikalischen Geschlecht eines bestimmten Begriffes sind Personen mit männlicher, weiblicher oder anderer Geschlechteridentität mit umfasst.

Die Erfindung betrifft ein Computertomographiegerät, aufweisend eine Gantry mit einer Öffnung und eine Strahlenschutzvorrichtung zur Abdeckung der Öffnung,
- wobei die Strahlenschutzvorrichtung eine Haltestruktur, einen Strahlenschutzvorhang und eine Schwenkvorrichtung aufweist,
- wobei ein Untersuchungsobjekt entlang einer Systemachse der Gantry in die Öffnung einführbar ist, wenn sich die Haltestruktur in einer ersten Position relativ zu der Gantry befindet,
- wobei die Haltestruktur mittels der Schwenkvorrichtung derart relativ zu der Gantry um eine Schwenkachse schwenkbar gelagert ist, dass durch eine erste Schwenkbewegung der Haltestruktur um die Schwenkachse die Haltestruktur relativ zu der Gantry von der ersten Position bis zu einer zweiten Position abgesenkt werden kann,
- wobei der Strahlenschutzvorhang an der Haltestruktur angeordnet ist, insbesondere an der Haltestruktur befestigt ist,
- wobei ein unterer Bereich des Strahlenschutzvorhangs von der Haltestruktur herabhängt.

Eine Ausführungsform sieht vor, dass ein oberer Bereich des Strahlenschutzvorhangs auf der Haltestruktur aufliegt.

Eine Ausführungsform sieht vor, dass der obere Bereich des Strahlenschutzvorhangs auf der Haltestruktur derart aufliegt, dass der obere Bereich des Strahlenschutzvorhangs einen Bereich einer Umgebung der Gantry vor Streustrahlen einer Röntgenstrahlung, die aus der Öffnung kommend auf den Bereich der Umgebung der Gantry gerichtet sind und die Haltestruktur durchdringen, abschirmt, insbesondere wenn sich die Haltestruktur in der zweiten Position der Haltestruktur befindet.

Eine Ausführungsform sieht vor, dass der untere Bereich des Strahlenschutzvorhangs und der obere Bereich des Strahlenschutzvorhangs entlang einer Kante des Strahlenschutzvorhangs aneinandergrenzen, wobei der Strahlenschutzvorhang derart an der Haltestruktur angeordnet ist, dass die Kante des Strahlenschutzvorhangs entlang eines ersten Rands der Haltestruktur verläuft.

Insbesondere kann vorgesehen sein, dass eine vertikale Position des ersten Rands der Haltestruktur in der zweiten Position der Haltestruktur tiefer ist als in der ersten Position der Haltestruktur. Der erste Rand der Haltestruktur kann sich insbesondere auf einer der Schwenkachse abgewandten Seite der Haltestruktur befinden. Die Schwenkachse kann sich beispielsweise in einem Bereich eines zweiten Rands der Haltestruktur befinden. Insbesondere kann vorgesehen sein, dass sich der obere Bereich des Strahlenschutzvorhangs von dem ersten Rand der Haltestruktur bis an die Schwenkachse und/oder bis zu dem zweiten Rand der Haltestruktur erstreckt.

Eine Ausführungsform sieht vor, dass der untere Bereich des Strahlenschutzvorhangs und der obere Bereich des Strahlenschutzvorhangs an der Kante des Strahlenschutzvorhangs miteinander vernäht sind. Der Strahlenschutzvorhang kann beispielsweise hergestellt werden, indem ein Stück, welches den unteren Bereich des Strahlenschutzvorhangs bildet, und ein Stück, welches den oberen Bereich des Strahlenschutzvorhangs bildet, miteinander an einer Stoßstelle, welche die Kante des Strahlenschutzvorhangs bildet, vernäht werden.

Eine Ausführungsform sieht vor, dass sich die Haltestruktur flächig erstreckt, wobei der obere Bereich des Strahlenschutzvorhangs auf der Haltestruktur flächig aufliegt. Eine Ausführungsform sieht vor, dass die Haltestruktur aus Kunststoff, insbesondere aus carbonfaserverstärktem Kunststoff, hergestellt ist. Damit kann die Haltestruktur gewichtsreduziert, stabil und einfach reinigbar ausgebildet sein. Die Haltestruktur kann beispielsweise in Form eines Deckels, insbesondere in Form eines vollflächig geschlossenen und/oder nach oben gewölbten Deckels, ausgebildet sein.

Eine Ausführungsform sieht vor, dass der Strahlenschutzvorhang mittels eines Satzes von Druckknopf-Verbindungen an der Haltestruktur befestigt ist. Damit kann der Strahlenschutzvorhang auf einfache Weise an der Haltestruktur sicher befestigt und/oder von der Haltestruktur gelöst werden.

Eine Ausführungsform sieht vor, dass der Strahlenschutzvorhang einen Satz von vorhangseitigen Verbindungselementen aufweist, wobei die Haltestruktur einen Satz von haltestrukturseitigen Verbindungselementen aufweist, wobei der Satz von vorhangseitigen Verbindungselementen und der Satz von haltestrukturseitigen Verbindungselementen zusammen den Satz von Druckknopf-Verbindungen bilden.

Insbesondere kann vorgesehen sein, dass der obere Bereich des Strahlenschutzvorhangs den Satz von vorhangseitigen Verbindungselementen aufweist und/oder dass eine Oberseite der Haltestruktur den Satz von haltestrukturseitigen Verbindungselementen aufweist, Insbesondere kann vorgesehen sein, dass die vorhangseitigen Verbindungselemente Druckknopf-Köpfe sind und/oder dass die haltestrukturseitigen Verbindungselemente Druckknopf-Vertiefungen zur Aufnahme der Druckknopf-Köpfe sind. Auch andere Zuordnungen von Druckknopf-Vertiefungen und Druckknopf-Köpfen sind grundsätzlich möglich.

Eine Ausführungsform sieht vor, dass der Satz von haltestrukturseitigen Verbindungselementen eine erste Reihe von haltestrukturseitigen Verbindungselementen aufweist, wobei die erste Reihe von haltestrukturseitigen Verbindungselementen einem Verlauf des ersten Rands der Haltestruktur folgend angeordnet ist, wobei der Satz von vorhangseitigen Verbindungselementen eine erste Reihe von vorhangseitigen Verbindungselementen aufweist, wobei die erste Reihe von vorhangseitigen Verbindungselementen einem Verlauf der Kante des Strahlenschutzvorhangs folgend und zu der ersten Reihe von haltestrukturseitigen Verbindungselementen korrespondierend angeordnet ist.

Insbesondere kann vorgesehen sein, dass der Satz von haltestrukturseitigen Verbindungselementen eine zweite Reihe von haltestrukturseitigen Verbindungselementen aufweist, wobei die zweite Reihe von haltestrukturseitigen Verbindungselementen einem Verlauf des zweiten Rands der Haltestruktur folgend angeordnet ist, wobei der Satz von vorhangseitigen Verbindungselementen eine zweite Reihe von vorhangseitigen Verbindungselementen aufweist, wobei die zweite Reihe von vorhangseitigen Verbindungselementen einem Verlauf der Kante des Strahlenschutzvorhangs folgend und zu der zweiten Reihe von haltestrukturseitigen Verbindungselementen korrespondierend angeordnet ist.

Eine Ausführungsform sieht vor, dass die Schwenkvorrichtung zumindest ein Friktionsscharnier aufweist, wobei die Haltestruktur mittels des zumindest einen Friktionsscharniers relativ zu der Gantry um die Schwenkachse schwenkbar gelagert ist, wobei das zumindest eine Friktionsscharnier dazu eingerichtet ist, die Haltestruktur relativ zu der Gantry in der zweiten Position zu halten, insbesondere indem ein Losbrechmoment des Friktionsscharniers größer ist als ein Drehmoment, welches durch ein Gewicht der Haltestruktur und des Strahlenschutzvorhangs in Bezug auf die Schwenkachse bewirkt wird.

Eine Ausführungsform sieht vor, dass die Schwenkvorrichtung einen gantryseitigen Bereich aufweist,
- wobei die Haltestruktur mittels der Schwenkvorrichtung relativ zu dem gantryseitigen Bereich um die Schwenkachse schwenkbar gelagert ist,
- wobei die Gantry einen Satz von Bolzen aufweist,
- wobei der gantryseitige Bereich einen Satz von Vertiefungen zur Aufnahme des Satzes von Bolzen aufweist,
- wobei der Satz von Bolzen derart in den Satz von Vertiefungen aufgenommen ist, dass der gantryseitige Bereich formschlüssig gegen eine Verschiebung relativ zu der Gantry in einer Ebene, die zu der Systemachse und/oder zu den Bolzen des Satzes von Bolzen im Wesentlichen senkrecht ist, gesichert ist.

Der gantryseitige Bereich kann beispielsweise im Wesentlichen stabförmig ausgebildet sein und/oder sich im Wesentlichen entlang der Schwenkachse erstrecken. Der gantryseitige Bereich kann beispielsweise im Wesentlichen aus Aluminium hergestellt sein.

Eine Ausführungsform sieht vor, dass die Bolzen des Satzes von Bolzen im Wesentlichen parallel zu der Systemachse ausgerichtet sind. Insbesondere kann vorgesehen sein, dass für jeden Bolzen des Satzes von Bolzen die Axialrichtung dieses Bolzens im Wesentlichen horizontal und/oder im Wesentlichen parallel zu der Systemachse ist.

Eine Ausführungsform sieht vor, dass zumindest ein Bolzen des Satzes von Bolzen eine Nut aufweist,
- wobei der gantryseitige Bereich in zumindest einer Vertiefung des Satzes von Vertiefungen ein Sicherungselement aufweist,
- wobei das Sicherungselement senkrecht zu einer Axialrichtung des zumindest einen Bolzens derart in die Nut eingeführt ist, dass der gantryseitige Bereich formschlüssig gegen eine Verschiebung relativ zu der Gantry in der Axialrichtung des zumindest einen Bolzens gesichert ist, insbesondere formschlüssig gegen eine Entfernung von der Gantry in der Axialrichtung des zumindest einen Bolzens gesichert ist.

Eine Ausführungsform sieht vor, dass der gantryseitige Bereich eine Feder aufweist, welche dazu eingerichtet ist, das Sicherungselement senkrecht zu der Axialrichtung des zumindest einen Bolzens gegen die Nut zu drücken.

Eine Ausführungsform sieht vor, dass der gantryseitige Bereich ein Bedienelement aufweist, welches an einer Oberfläche des gantryseitigen Bereichs angeordnet ist und mit dem Sicherungselement derart verbunden ist, dass durch einen Druck auf das Bedienelement ein Herausdrücken des Sicherungselements aus der Nut bewirkt werden kann, insbesondere entgegen der Federkraft der Feder bewirkt werden kann. Der Druck auf das Bedienelement kann beispielsweise manuell und/oder durch einen Finger einer Bedienperson erfolgen.

Die Haltestruktur kann insbesondere formstabil ausgebildet sein. Die Schwenkachse kann beispielsweise im Wesentlichen senkrecht zu der Systemachse sein, insbesondere senkrecht zu der Systemachse sein. Die Schwenkachse kann beispielsweise im Wesentlichen parallel zu der Systemachse sein, insbesondere parallel zu der Systemachse sein. Die Schwenkachse kann beispielsweise windschief zu der Systemachse sein und dabei insbesondere weder im Wesentlichen senkrecht noch im Wesentlichen parallel zu der Systemachse sein.

Die Öffnung kann beispielsweise tunnelförmig sein und/oder sich entlang der Systemachse erstrecken. Die Systemachse kann beispielsweise durch die Öffnung hindurch verlaufen, insbesondere durch einen zentralen Bereich der Öffnung verlaufen. Die Systemachse kann beispielsweise durch ein Isozentrum des Computertomographiegeräts verlaufen. Insbesondere kann vorgesehen sein, dass die vertikale Position des ersten Rands der Haltestruktur sowohl in der ersten Position der Haltestruktur als auch in der zweiten Position der Haltestruktur höher ist als eine vertikale Position der Systemachse.

Die Schwenkvorrichtung kann insbesondere ein Schwenklager sein und/oder in Form eines Klappmechanismus ausgebildet sein. Die Strahlenschutzvorrichtung ist so gestaltet, dass, wenn sich die Haltestruktur in der ersten Position relativ zu der Gantry befindet, ein Patient relativ zu der Gantry derart positioniert werden kann, dass sich der Kopf des Patienten in der Öffnung befindet, und dass, wenn sich die Haltestruktur in der zweiten Position relativ zu der Gantry befindet, die Haltestruktur den Patienten nicht berührt.

Eine solche Strahlenschutzvorrichtung ist von beiden Randbereichen links bzw. rechts neben dem Patientenbett, in denen sich das Bedienpersonal für die Untersuchung und/oder für die Vorbereitung der Untersuchung aufhält, gut erreichbar, insbesondere sowohl für das Absenken der Haltestruktur aus der ersten Position als auch für das Anheben der Haltestruktur aus der zweiten Position.

Weiterhin kann vorgesehen sein, dass die Haltestruktur mittels der Schwenkvorrichtung derart relativ zu der Gantry um die Schwenkachse schwenkbar gelagert ist, dass durch eine zweite Schwenkbewegung der Haltestruktur um die Schwenkachse der die Haltestruktur relativ zu der Gantry von der zweiten Position bis zu der ersten Position angehoben werden kann.

Eine Ausführungsform sieht vor, dass die Gantry einen Innenbereich und eine Verkleidung zum Abgrenzen des Innenbereichs von einer Umgebung aufweist, wobei die Haltestruktur an einem Bereich der Verkleidung anliegt, wenn sich die Haltestruktur in der ersten Position der Haltestruktur befindet, wobei die Haltestruktur von dem Bereich der Verkleidung wegragt, wenn sich die Haltestruktur in der zweiten Position der Haltestruktur befindet.

Eine Ausführungsform sieht vor, dass sich die Haltestruktur flächig in einer Deckelebene erstreckt und dass die Deckelebene zu der Schwenkachse im Wesentlichen parallel ist. Beispielsweise kann ein Winkel zwischen den Deckelebene und der Systemachse größer als 30 Grad und/oder kleiner als 60 Grad, insbesondere ungefähr 45 Grad, sein, wenn sich die Haltestruktur in der zweiten Position der Haltestruktur befindet. Eine andere Ausführungsform sieht vor, dass sich die Haltestruktur flächig in einer Deckelebene erstreckt und dass sich die Deckelebene und die Schwenkachse unter einem Winkel schneiden, der deutlich von Null verschieden ist, beispielsweise ungefähr 45 Grad ist.

Die Schwenkachse kann sich beispielsweise in einem Bereich eines zweiten Rands der Haltestruktur befinden. Der erste Rand der Haltestruktur und der zweite Rand der Haltestruktur können insbesondere einander in Bezug auf einen zentralen Bereich der Haltestruktur gegenüberliegend angeordnet sein. Der Abstand zwischen dem ersten Rand der Haltestruktur und der Schwenkachse kann beispielsweise größer als 10 cm, insbesondere größer als 20 cm, insbesondere größer als 30 cm sein. Der Abstand zwischen dem ersten Rand der Haltestruktur und einem zweiten Rand der Haltestruktur kann beispielsweise größer als 10 cm, insbesondere größer als 20 cm, insbesondere größer als 30 cm sein.

Weiterhin kann vorgesehen sein, dass die Haltestruktur über die zweite Position hinaus weiter abgesenkt werden kann, beispielsweise derart, dass die Haltestruktur eine Position relativ zu der Gantry einnehmen kann, in welcher die Deckelebene im Wesentlichen senkrecht, insbesondere senkrecht, zu der Systemachse ist und/oder in welcher die Haltestruktur an einem Rand der Öffnung anliegt. Das kann beispielsweise für eine Kalibrierungsmessung hilfreich sein, bei welcher ein Untersuchungsobjekt in Form eines Phantoms in der Öffnung angeordnet werden kann, ohne dass mit dem Untersuchungsobjekt verbundene Teile aus der Öffnung herausragen.

Eine Ausführungsform sieht vor, dass die Systemachse im Wesentlichen horizontal, insbesondere horizontal, ist und dass die Schwenkachse im Wesentlichen horizontal, insbesondere horizontal, ist. Eine Ausführungsform sieht vor, dass sich die Schwenkachse oberhalb der Öffnung befindet.

Insbesondere kann vorgesehen sein, dass die vertikale Position des ersten Rands der Haltestruktur in der ersten Position der Haltestruktur höher ist als eine vertikale Position der Schwenkachse und/oder dass die vertikale Position des ersten Rands der Haltestruktur in der zweiten Position der Haltestruktur tiefer ist als eine vertikale Position der Schwenkachse.

Mit Hilfe des Strahlenschutzvorhangs können beispielsweise Lücken, welche sich zwischen der Verkleidung der Gantry einerseits und dem Patienten und/oder des Patientenbetts andererseits befinden, abgedeckt werden. Der Strahlenschutzvorhang kann insbesondere ein flexibles flächiges Strahlenschutzmaterial aufweisen und/oder für Streustrahlen der in dem Computertomographiegerät zur Untersuchung des Untersuchungsobjekts verwendeten Röntgenstrahlung im Wesentlichen undurchlässig sein. Das flexible flächige Strahlenschutzmaterial kann beispielsweise bleifrei oder bleihaltig sein. Der Strahlenschutzvorhang kann beispielsweise einen für sichtbares Licht transparenten Bereich, beispielsweise in Form eines Bleiglasfensters, aufweisen.

Eine Ausführungsform sieht vor, dass sich ein erster seitlicher Teil des Strahlenschutzvorhangs von der Gantry bis zu einem ersten Rand der Haltestruktur erstreckt, insbesondere im Wesentlichen parallel zu der Systemachse erstreckt, und dass sich ein zweiter seitlicher Teil des Strahlenschutzvorhangs von der Gantry bis zu dem ersten Rand der Haltestruktur erstreckt, insbesondere im Wesentlichen parallel zu der Systemachse erstreckt.

Weiterhin kann vorgesehen sein, dass sich ein vorderer Teil des Strahlenschutzvorhangs von dem ersten seitlichen Teil des Strahlenschutzvorhangs bis zu dem zweiten seitlichen Teil des Strahlenschutzvorhangs erstreckt, insbesondere im Wesentlichen senkrecht zu der Systemachse erstreckt. Weiterhin kann vorgesehen sein, dass sich die Systemachse zwischen dem ersten seitlichen Teil des Strahlenschutzvorhangs und dem zweiten seitlichen Teil des Strahlenschutzvorhangs befindet, wenn sich die Haltestruktur in der zweiten Position der Haltestruktur befindet.

Durch das Absenken und das Anheben der Haltestruktur wird der Strahlenschutzvorhang im Wesentlichen vertikal relativ zu dem Patienten bewegt. Dadurch ist die Gefahr verringert, dass mit dem Patienten verbundene Kabel und/oder Schläuche von dem Strahlenschutzvorhang mitgerissen und beschädigt werden. Durch die Befestigung an der Haltestruktur kann der Strahlenschutzvorhang derart relativ zu dem Patienten positioniert werden, dass möglichst wenig Gewicht des Strahlenschutzvorhangs auf dem Patienten lastet und dass das auf dem Patienten lastende Gewicht des Strahlenschutzvorhangs möglichst gleichmäßig über die betroffenen Bereiche des Patienten verteilt ist.

Eine Ausführungsform sieht vor, dass die Gantry einen ersten Gantryteil, einen zweiten Gantryteil und einen dritten Gantryteil aufweist, wobei der erste Gantryteil einen drehbar gelagerten Rotor mit einem Projektionsdatenakquisitionssystem aufweist, wobei der dritte Gantryteil zumindest einen Abschnitt der Öffnung aufweist, wobei die Haltestruktur mittels der Schwenkvorrichtung mit dem dritten Gantryteil verbunden und relativ zu dem dritten Gantryteil um die Schwenkachse schwenkbar gelagert ist. Das Projektionsdatenakquisitionssystem kann beispielsweise eine Röntgenquelle und einen Röntgendetektor, welcher mit der Röntgenquelle zusammenwirkt, aufweisen.

Dabei kann der erste Gantryteil relativ zu dem zweiten Gantryteil und relativ zu dem dritten Gantryteil derart bewegbar gelagert sein, dass eine Translationsbewegung des ersten Gantryteils relativ zu dem zweiten Gantryteil und relativ zu dem dritten Gantryteil ausgeführt werden kann, während gleichzeitig dazu der zweite Gantryteil und der dritte Gantryteil relativ zu dem Untersuchungsobjekt ruhen und die Strahlenschutzvorrichtung relativ zu dem Untersuchungsobjekt und relativ zu dem zumindest einen Abschnitt der Öffnung ruht, wenn sich das Untersuchungsobjekt in der Öffnung befindet.

Das Untersuchungsobjekt kann beispielsweise ein Körperteil eines Patienten, insbesondere ein Kopf eines Patienten, sein. Das Computertomographiegerät kann insbesondere als Kopf-Computertomographiegerät und/oder als mobiles Computertomographiegerät ausgebildet sein.

Bei dem Patienten kann es sich beispielsweise um einen Menschen, insbesondere um ein Baby, oder um ein Tier handeln. Eine Längsachse des Patienten kann beispielsweise zu der Systemachse parallel, insbesondere identisch, oder windschief sein oder die Systemachse schneiden.

Das Untersuchungsobjekt kann beispielsweise ein Gegenstand, insbesondere ein Phantom zur Kalibrierung des Computertomographiegeräts, sein.

Eine Ausführungsform sieht vor, dass der erste Gantryteil eine Drehlagerung und eine Tragstruktur aufweist, wobei der Rotor mittels der Drehlagerung mit der Tragstruktur verbunden und relativ zu der Tragstruktur um die Systemachse drehbar gelagert ist. Das zweite Gantryteil kann beispielsweise ein Fahrwerk und/oder eine Kopfschale aufweisen.

Das Computertomographiegerät kann beispielsweise ferner ein Beleuchtungssystem aufweisen, welches dazu eingerichtet ist, die Öffnung zumindest dann zu beleuchten, wenn sich die Haltestruktur in der zweiten Position der Haltestruktur befindet.

Das Beleuchtungssystem kann beispielsweise eine haltestrukturseitige Lichtquelle aufweisen, welche an der Haltestruktur derart befestigt ist, dass die Lichtquelle die Öffnung beleuchtet, wenn sich die Haltestruktur in der zweiten Position relativ zu der Gantry befindet. Das Beleuchtungssystem kann beispielsweise eine rückseitige Lichtquelle aufweisen, welche an einem Strahlenschutzkörper, der die Rückseite der Öffnung verschließt, befestigt ist.

Mit Hilfe des Beleuchtungssystems kann eine ausreichende Beleuchtung der Öffnung auch dann ermöglicht werden, wenn die Rückseite der Öffnung durch einen für sichtbares Licht undurchlässigen Strahlenschutzkörper verschlossen ist.

Das Computertomographiegerät kann beispielsweise ferner ein Kamerasystem aufweisen, welches dazu eingerichtet ist, das Untersuchungsobjekt zumindest dann optisch zu erfassen, wenn sich das Untersuchungsobjekt in der Öffnung befindet und wenn sich die Haltestruktur in der zweiten Position der Haltestruktur befindet.

Die Verwendung des Kamerasystems innerhalb des Strahlenschutzbereiches, beispielsweise innerhalb der Öffnung, ermöglicht eine Patientenbeobachtung auch dann, wenn sichtbares Licht nicht aus der Öffnung nach außen dringen kann oder wenn eine Patientenbeobachtung durch einen für sichtbares Licht transparenten Bereich der Haltestruktur hindurch nicht erfolgen kann. Das Computertomographiegerät kann insbesondere ein Display aufweisen. Ein von dem Kamerasystem erzeugtes Bild des Untersuchungsobjekts kann beispielsweise auf dem Display angezeigt werden.

Das Kamerasystem kann beispielsweise eine an der Gantry befestigte Kamera und/oder eine an der Haltestruktur befestigte Kamera und/oder eine an einem Strahlenschutzkörper, der die Rückseite der Öffnung verschließt, befestigte Kamera aufweisen. Die an der Gantry befestige Kamera kann insbesondere an einem Teil der Verkleidung der Gantry, welcher die Öffnung begrenzt, befestigt sein. Weiterhin kann vorgesehen sein, dass das Kamerasystem wenigstens eine Kameralichtquelle aufweist und damit das Beleuchtungssystem bildet. Die Kameralichtquelle kann beispielsweise einen Kranz aus Leuchtdioden, welcher ein Objektiv der Kamera ringförmig umgibt, aufweisen.

Das Computertomographiegerät kann beispielsweise ferner ein akustisches System aufweisen, welches dazu eingerichtet ist, ein akustisches Signal an das Untersuchungsobjekt zumindest dann zu senden und/oder ein von dem Untersuchungsobjekt ausgehendes akustisches Signal zumindest dann zu empfangen, wenn sich das Untersuchungsobjekt in der Öffnung befindet und wenn sich die Haltestruktur in der zweiten Position der Haltestruktur befindet.

Das akustische System kann beispielsweise ein Mikrofon und/oder einen Lautsprecher aufweisen. Das Mikrofon kann beispielsweise an der Gantry oder an der Haltestruktur oder an einem Strahlenschutzkörper, der die Rückseite der Öffnung verschließt, befestigt sein. Der Lautsprecher kann beispielsweise an der Gantry oder an der Haltestruktur oder an einem Strahlenschutzkörper, der die Rückseite der Öffnung verschließt, befestigt sein.

Das Computertomographiegerät kann beispielsweise ferner ein optisches System aufweisen, welches dazu eingerichtet ist, ein optisches Signal an das Untersuchungsobjekt zumindest dann zu senden, wenn sich das Untersuchungsobjekt in der Öffnung befindet und wenn sich die Haltestruktur in der zweiten Position der Haltestruktur befindet.

Das optische System kann beispielsweise ein Display zur Anzeige von optischen Signalen in Form von Texten und/oder Bildern und/oder einen Projektor zur Projektion von optischen Signalen in Form von Lichtstrahlen, insbesondere Laserstrahlen, aufweisen. Das Display kann beispielsweise an der Gantry oder an der Haltestruktur oder an einem Strahlenschutzkörper, der die Rückseite der Öffnung verschließt, befestigt sein. Der Projektor kann beispielsweise an der Gantry oder an der Haltestruktur oder an einem Strahlenschutzkörper, der die Rückseite der Öffnung verschließt, befestigt sein.

Weiterhin kann das Computertomographiegerät einen Strahlenschutzkörper aufweisen, welcher mit der Gantry derart lösbar verbunden werden kann, dass er eine Rückseite der Öffnung abdeckt. Die Rückseite der Öffnung kann sich insbesondere auf einer Rückseite der Gantry befinden, wobei eine Vorderseite der Gantry der Strahlenschutzvorrichtung zugewandt ist. Insbesondere kann vorgesehen sein, dass der erste Gantryteil die Rückseite der Öffnung aufweist und dass der dritte Gantryteil die Vorderseite der Öffnung aufweist. Der Strahlenschutzkörper kann beispielsweise eine Schnittstelle zur Energieversorgung und/oder eine Schnittstelle zur Datenübertragung für wenigstens eine Komponente des Beleuchtungssystems, des Kamerasystems, des akustischen Systems und/oder des optischen Systems aufweisen.

Im Rahmen der Erfindung können Merkmale, welche in Bezug auf unterschiedliche Ausführungsformen der Erfindung und/oder unterschiedliche Anspruchskategorien (Verfahren, Verwendung, Vorrichtung, System, Anordnung usw.) beschrieben sind, zu weiteren Ausführungsformen der Erfindung kombiniert werden. Beispielsweise kann ein Anspruch, der eine Vorrichtung betrifft, auch mit Merkmalen, die im Zusammenhang mit einem Verfahren beschrieben oder beansprucht sind, weitergebildet werden und umgekehrt. Funktionale Merkmale eines Verfahrens können dabei durch entsprechend ausgebildete gegenständliche Komponenten ausgeführt werden. Die Verwendung der unbestimmten Artikel "ein" bzw. "eine" schließt nicht aus, dass das betroffene Merkmal auch mehrfach vorhanden sein kann.

Im Folgenden werden Merkmale der Erfindung anhand von Beispielen unter Hinweis auf die beigefügten Figuren erläutert. Die Darstellung in den Figuren ist schematisch, stark vereinfacht und nicht zwingend maßstabsgetreu.

Die Fig. 1 zeigt ein Computertomographiegerät mit einer Gantry und einer Strahlenschutzvorrichtung zur Abdeckung einer Öffnung.

Die Figuren 2 bis 9 zeigen verschiedene Ansichten von Teilen der Strahlenschutzvorrichtung und/oder der Gantry.

Die Fig. 10 zeigt ein Computertomographiegerät mit einer Gantry und einer Strahlenschutzvorrichtung zur Abdeckung einer Öffnung in einer weiteren Ansicht.

Die Fig. 11 zeigt ein Computertomographiegerät mit einer Gantry und einer Strahlenschutzvorrichtung zur Abdeckung einer Öffnung in einer weiteren Ansicht.

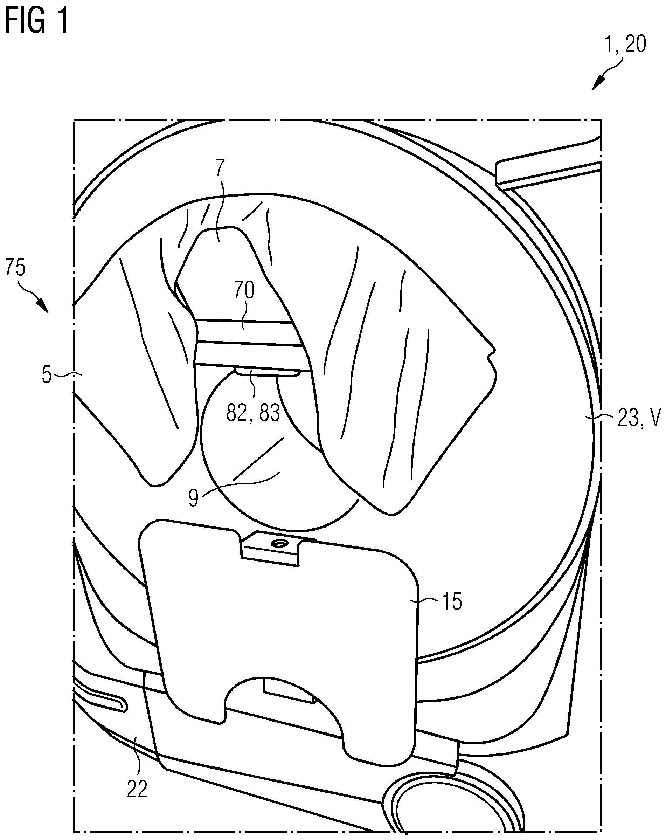

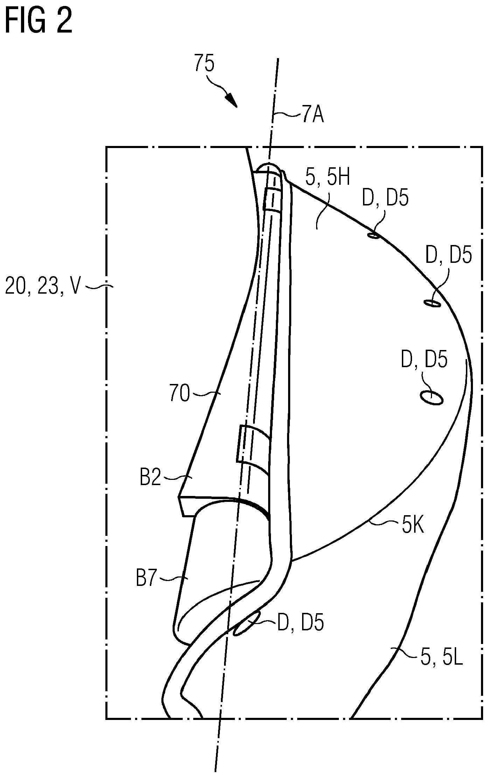

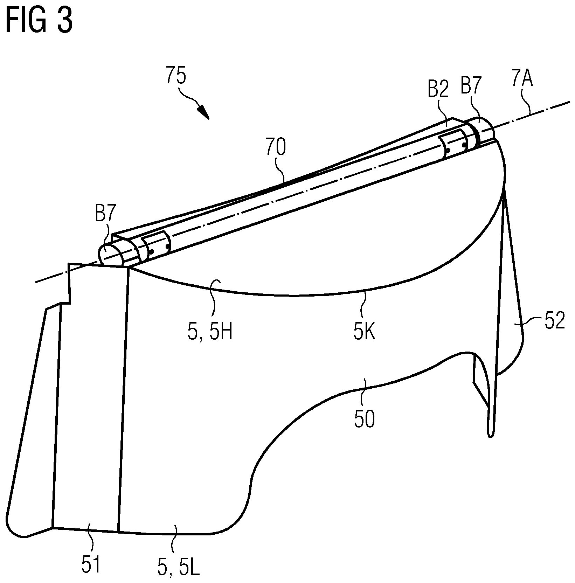

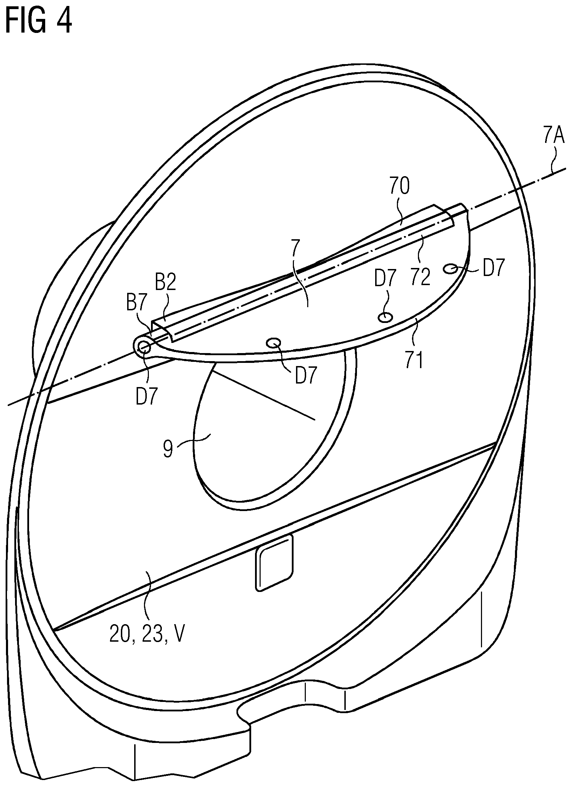

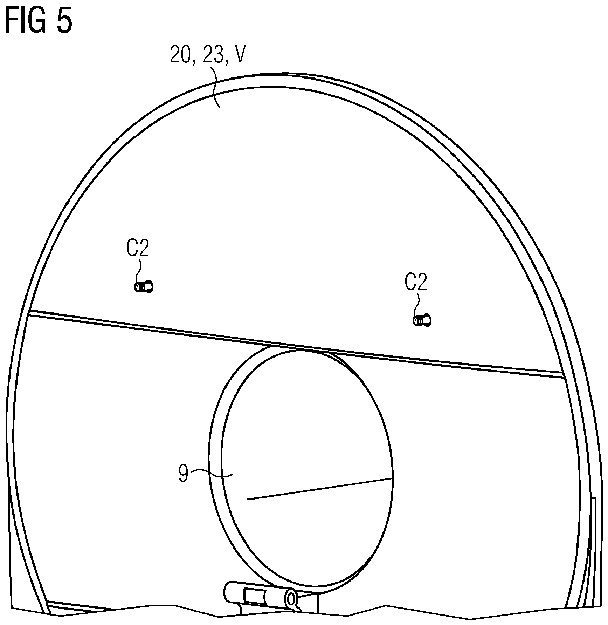

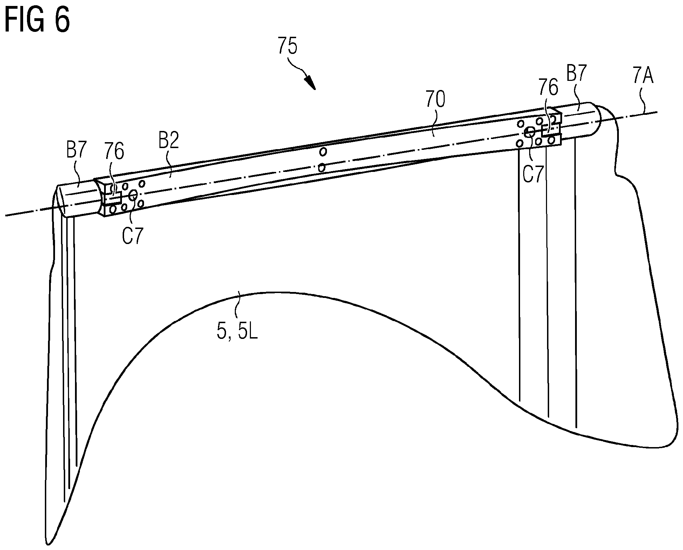

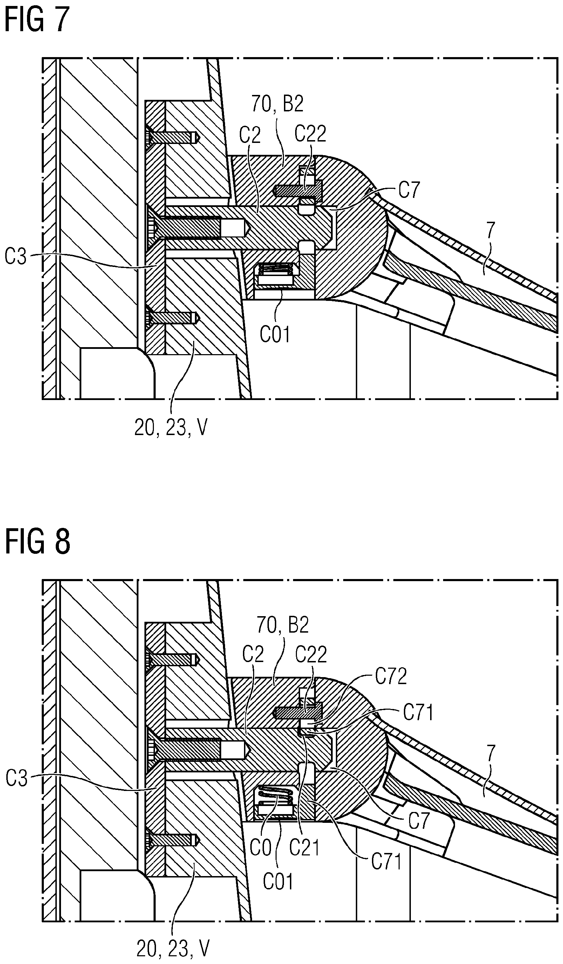

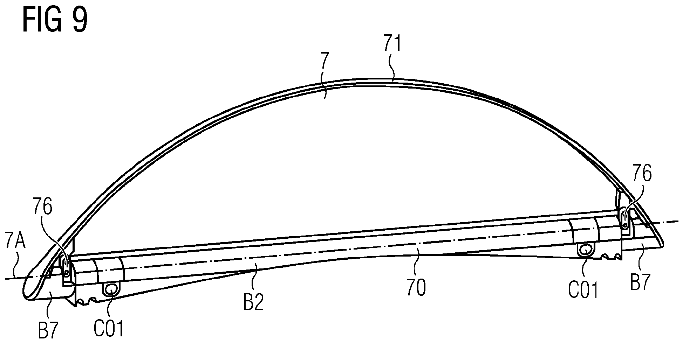

Die Fig. 1 zeigt das Computertomographiegerät 1 mit der Gantry 20 und der Strahlenschutzvorrichtung 75 zur Abdeckung der Öffnung 9, wobei sich die Haltestruktur 7 in der ersten Position befindet. Die Figuren 2 bis 9 zeigen verschiedene Ansichten von Teilen der Strahlenschutzvorrichtung 75 und/oder der Gantry 20.

Durch die vorkragende Haltestruktur 7 in der zweiten Position wird entsprechender Raum unterhalb des Strahlenschutzvorhangs 5 geschaffen. Es gibt somit unter dem Strahlenschutzvorhang 5 mehr Platz für beispielsweise einen Tubus, Schläuche und ähnliches Zubehör für die medizinische Bildgebungsuntersuchung.

Die Strahlenschutzvorrichtung 75 weist den Strahlenschutzvorhang 5 auf, wobei der Strahlenschutzvorhang 5 an der Haltestruktur 7 befestigt ist und von der Haltestruktur 7 herabhängt. Ein erster seitlicher Teil 51 des Strahlenschutzvorhangs 5 erstreckt sich von der Gantry 20 bis zu einem ersten Rand 71 der Haltestruktur 7. Ein zweiter seitlicher Teil 52 des Strahlenschutzvorhangs 5 von der Gantry 20 erstreckt sich bis zu dem ersten Rand 71 der Haltestruktur 7. Ein vorderer Teil 50 des Strahlenschutzvorhangs 5 erstreckt sich von dem ersten seitlichen Teil 51 des Strahlenschutzvorhangs 5 bis zu dem zweiten seitlichen Teil 52 des Strahlenschutzvorhangs 5. Die Systemachse SA befindet sich zwischen dem ersten seitlichen Teil 51 des Strahlenschutzvorhangs 5 und dem zweiten seitlichen Teil 52 des Strahlenschutzvorhangs 5, wenn sich die Haltestruktur 7 in der zweiten Position der Haltestruktur 7 befindet.

Die Schwenkachse 7A befindet sich in einem Bereich eines zweiten Rands 72 der Haltestruktur 7. Der erste Rand 71 und der zweite Rand 72 sind einander in Bezug auf einen zentralen Bereich der Haltestruktur 7 gegenüberliegend angeordnet.

Das Computertomographiegerät 1 gemäß dem gezeigten Beispiel weist eine Gantry 20 mit einer Öffnung 9 und eine Strahlenschutzvorrichtung 75 zur Abdeckung der Öffnung 9 auf,
- wobei die Strahlenschutzvorrichtung 75 eine Haltestruktur 7, einen Strahlenschutzvorhang 5 und eine Schwenkvorrichtung 70 aufweist,
- wobei ein Untersuchungsobjekt 14 entlang einer Systemachse SA der Gantry 20 in die Öffnung 9 einführbar ist, wenn sich die Haltestruktur 7 in einer ersten Position relativ zu der Gantry 20 befindet,
- wobei die Haltestruktur 7 mittels der Schwenkvorrichtung 70 derart relativ zu der Gantry 20 um eine Schwenkachse 7A schwenkbar gelagert ist, dass durch eine erste Schwenkbewegung der Haltestruktur 7 um die Schwenkachse 7A die Haltestruktur 7 relativ zu der Gantry 20 von der ersten Position bis zu einer zweiten Position abgesenkt werden kann,
- wobei der Strahlenschutzvorhang 5 an der Haltestruktur 7 angeordnet ist, insbesondere an der Haltestruktur 7 befestigt ist,
- wobei ein unterer Bereich 5L des Strahlenschutzvorhangs 5 von der Haltestruktur 7 herabhängt,
- wobei ein oberer Bereich 5H des Strahlenschutzvorhangs 5 auf der Haltestruktur 7 aufliegt.

Das gezeigte Beispiel sieht vor, dass der obere Bereich 5H des Strahlenschutzvorhangs 5 auf der Haltestruktur 7 derart aufliegt, dass der obere Bereich 5H des Strahlenschutzvorhangs 5 einen Bereich 8 einer Umgebung U der Gantry 20 vor Streustrahlen einer Röntgenstrahlung 4, die aus der Öffnung 9 kommend auf den Bereich 8 der Umgebung U der Gantry 20 gerichtet sind und die Haltestruktur 7 durchdringen, abschirmt, insbesondere wenn sich die Haltestruktur 7 in der zweiten Position der Haltestruktur 7 befindet.

Das gezeigte Beispiel sieht vor, dass der untere Bereich 5L des Strahlenschutzvorhangs 5 und der obere Bereich 5H des Strahlenschutzvorhangs 5 entlang einer Kante 5K des Strahlenschutzvorhangs 5 aneinandergrenzen, - wobei der Strahlenschutzvorhang 5 derart an der Haltestruktur 7 angeordnet ist, dass die Kante 5K des Strahlenschutzvorhangs 5 entlang eines ersten Rands 71 der Haltestruktur 7 verläuft.

Insbesondere kann vorgesehen sein, dass eine vertikale Position des ersten Rands 71 der Haltestruktur 7 in der zweiten Position der Haltestruktur 7 tiefer ist als in der ersten Position der Haltestruktur 7. Der erste Rand 71 der Haltestruktur 7 kann sich insbesondere auf einer der Schwenkachse 7A abgewandten Seite der Haltestruktur 7 befinden. Die Schwenkachse 7A kann sich beispielsweise in einem Bereich eines zweiten Rands 72 der Haltestruktur 7 befinden. Insbesondere kann vorgesehen sein, dass sich der obere Bereich 5H des Strahlenschutzvorhangs 5 von dem ersten Rand 71 der Haltestruktur 7 bis an die Schwenkachse 7A und/oder bis zu dem zweiten Rand 72 der Haltestruktur 7 erstreckt.

Das gezeigte Beispiel sieht vor, dass der untere Bereich 5L des Strahlenschutzvorhangs 5 und der obere Bereich 5H des Strahlenschutzvorhangs 5 an der Kante 5K des Strahlenschutzvorhangs 5 miteinander vernäht sind. Das gezeigte Beispiel sieht vor, dass sich die Haltestruktur 7 flächig erstreckt, wobei der obere Bereich 5H des Strahlenschutzvorhangs 5 auf der Haltestruktur 7 flächig aufliegt. Das gezeigte Beispiel sieht vor, dass die Haltestruktur 7 aus Kunststoff, insbesondere aus carbonfaserverstärktem Kunststoff, hergestellt ist.

Das gezeigte Beispiel sieht vor, dass der Strahlenschutzvorhang 5 mittels eines Satzes von Druckknopf-Verbindungen D an der Haltestruktur 7 befestigt ist. Das gezeigte Beispiel sieht vor, dass der Strahlenschutzvorhang 5 einen Satz von vorhangseitigen Verbindungselementen D5 aufweist, - wobei die Haltestruktur 7 einen Satz von haltestrukturseitigen Verbindungselementen D7 aufweist, wobei der Satz von vorhangseitigen Verbindungselementen D5 und der Satz von haltestrukturseitigen Verbindungselementen D7 zusammen den Satz von Druckknopf-Verbindungen D bilden.

Das gezeigte Beispiel sieht vor, dass der Satz von haltestrukturseitigen Verbindungselementen D7 eine erste Reihe von haltestrukturseitigen Verbindungselementen D7 aufweist, wobei die erste Reihe von haltestrukturseitigen Verbindungselementen D7 einem Verlauf des ersten Rands 71 der Haltestruktur 7 folgend angeordnet ist, wobei der Satz von vorhangseitigen Verbindungselementen D5 eine erste Reihe von vorhangseitigen Verbindungselementen D5 aufweist, wobei die erste Reihe von vorhangseitigen Verbindungselementen D5 einem Verlauf der Kante 5K des Strahlenschutzvorhangs 5 folgend und zu der ersten Reihe von haltestrukturseitigen Verbindungselementen D7 korrespondierend angeordnet ist.

Das gezeigte Beispiel sieht vor, dass die Schwenkvorrichtung 70 zumindest ein Friktionsscharnier 76 aufweist,
- wobei die Haltestruktur 7 mittels des zumindest einen Friktionsscharniers 76 relativ zu der Gantry 20 um die Schwenkachse 7A schwenkbar gelagert ist,
- wobei das zumindest eine Friktionsscharnier 76 dazu eingerichtet ist, die Haltestruktur 7 relativ zu der Gantry 20 in der zweiten Position zu halten, insbesondere indem ein Losbrechmoment des Friktionsscharniers 76 größer ist als ein Drehmoment, welches durch ein Gewicht der Haltestruktur 7 und des Strahlenschutzvorhangs 5 in Bezug auf die Schwenkachse 7A bewirkt wird.

Das gezeigte Beispiel sieht vor, dass die Schwenkvorrichtung 70 einen gantryseitigen Bereich B2 aufweist,
- wobei die Haltestruktur 7 mittels der Schwenkvorrichtung 70 relativ zu dem gantryseitigen Bereich B2 um die Schwenkachse 7A schwenkbar gelagert ist,
- wobei die Gantry 20 einen Satz von Bolzen C2 aufweist,
- wobei der gantryseitige Bereich B2 einen Satz von Vertiefungen C7 zur Aufnahme des Satzes von Bolzen C2 aufweist,
- wobei der Satz von Bolzen C2 derart in den Satz von Vertiefungen C7 aufgenommen ist, dass der gantryseitige Bereich B2 formschlüssig gegen eine Verschiebung relativ zu der Gantry 20 in einer Ebene, die zu der Systemachse SA und/oder zu den Bolzen des Satzes von Bolzen C2 im Wesentlichen senkrecht ist, gesichert ist.

Der gantryseitige Bereich B2 kann beispielsweise im Wesentlichen stabförmig ausgebildet sein und/oder sich im Wesentlichen entlang der Schwenkachse 7A erstrecken. Der gantryseitige Bereich B2 kann beispielsweise im Wesentlichen aus Aluminium hergestellt sein.

Das gezeigte Beispiel sieht vor, dass die Bolzen des Satzes von Bolzen C2 im Wesentlichen parallel zu der Systemachse SA ausgerichtet sind.

Das gezeigte Beispiel sieht vor, dass zumindest ein Bolzen des Satzes von Bolzen C2 eine Nut C21 aufweist, - wobei der gantryseitige Bereich B2 in zumindest einer Vertiefung des Satzes von Vertiefungen C7 ein Sicherungselement C71 aufweist, wobei das Sicherungselement C71 senkrecht zu einer Axialrichtung des zumindest einen Bolzens derart in die Nut C21 eingeführt ist, dass der gantryseitige Bereich B2 formschlüssig gegen eine Verschiebung relativ zu der Gantry 20 in der Axialrichtung des zumindest einen Bolzens gesichert ist, insbesondere formschlüssig gegen eine Entfernung von der Gantry 20 in der Axialrichtung des zumindest einen Bolzens gesichert ist.

Das gezeigte Beispiel sieht vor, dass der gantryseitige Bereich B2 eine Feder C0 aufweist, welche dazu eingerichtet ist, das Sicherungselement C71 senkrecht zu der Axialrichtung des zumindest einen Bolzens gegen die Nut C21 zu drücken.

Das gezeigte Beispiel sieht vor, dass der gantryseitige Bereich B2 ein Bedienelement C01 aufweist, welches an einer Oberfläche des gantryseitigen Bereichs B2 angeordnet ist und mit dem Sicherungselement C71 derart verbunden ist, dass durch einen Druck auf das Bedienelement C01 ein Herausdrücken des Sicherungselements C71 aus der Nut C21 bewirkt werden kann, insbesondere entgegen der Federkraft der Feder bewirkt werden kann.

Die Metallplatte C3 ist von innen an die Verkleidung V geschraubt. Jeder Bolzen des Satzes von Bolzen C2 ist mit einer entsprechenden Metallplatte C3 verschraubt. Das Sicherungselement C71 umfasst einen plattenförmigen Bereich mit entsprechenden Löchern rastet in der Nut C21 ein. Die Feder C0 hält das Sicherungselement C71 in der Nut C21. Der gantryseitige Bereich B2 ist somit relativ zu der Gantry 20 fixiert. Zu Reinigungszwecken und/oder für eine Verwendung der Gantry 20 ohne die Strahlenschutzvorrichtung 75, beispielsweise innerhalb eines Krankenwagens, kann die Strahlenschutzvorrichtung 75 so auf einfache Weise von der Gantry 20 gelöst werden.

Der plattenförmige Bereich des Sicherungselements C71 erstreckt sich flächig in einer zu der Axialrichtung des Bolzens C2 senkrechen Ebene und weist ein kreisrundes Loch auf, durch das sich der Bolzen C2 hindurch erstreckt. Der plattenförmige Bereich des Sicherungselements C71 weist ferner das Langloch C72 auf, welches sich senkrecht zu der Axialrichtung des Bolzens C2 erstreckt. Die Schraube C22 erstreckt sich durch das Langloch C72 hindurch und bildet zusammen mit dem Langloch C72 eine Führung für das Sicherungselement C71.

Der gantryseitige Bereich B2 ist mit dem gantryseitigen Teil des zumindest einen Friktionsscharniers 76 verbunden. Der haltestrukturseitige Teil des zumindest einen Friktionsscharniers 76 kann beispielsweise mit einer Gewindeplatte verschraubt sein, welche in die Haltestruktur 7 einlaminiert ist, insbesondere in ein entsprechendes Endstück B7 der Haltestruktur 7 einlaminiert ist. An jedem der beiden Endstücke B7 der Haltestruktur 7 ist jeweils ein haltestrukturseitiges Verbindungselement des Satzes von haltestrukturseitigen Verbindungselementen D7 befestigt.

Das zumindest eine Friktionsscharnier 76 sorgt für genügend Haltekraft, damit die Haltestruktur 7 in diversen Positionen, insbesondere in jeder Position, die bei der ersten Schwenkbewegung von der ersten Position bis zu der zweiten Position durchlaufen wird, relativ zu der Gantry 20 gehalten werden kann. Somit kann die Haltestruktur 7 in unterschiedlichen Winkelstellungen relativ zu der Gantry 20 positioniert werden. Insbesondere kann vorgesehen sein, dass das zumindest eine Friktionsscharnier 76 die Haltestruktur 7 in jeder Position zwischen 90 Grad nach oben relativ zu der Systemachse SA und 90 Grad nach unten relativ zu der Systemachse SA halten kann.

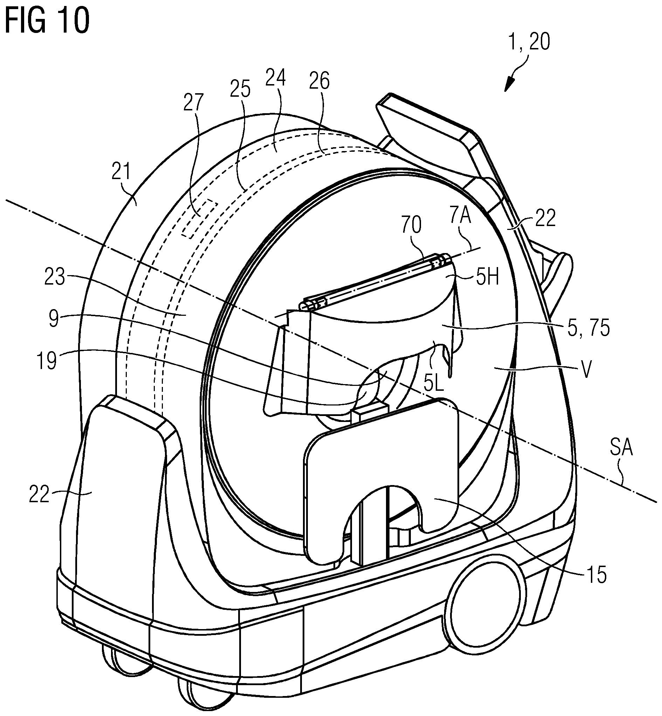

Die Fig. 10 zeigt ein Computertomographiegerät mit einer Gantry und einer Strahlenschutzvorrichtung zur Abdeckung einer Öffnung in einer weiteren Ansicht, wobei sich die Haltestruktur 7 in der zweiten Position befindet. Das Untersuchungsobjekt 14 ist der Kopf des Patienten 13. Das Computertomographiegerät 1 ist ein mobiles Kopf-Computertomographiegerät. Das Computertomographiegerät 1 weist ferner eine Kopfschale 19, in welche der Kopf des Patienten 13 aufgenommen werden kann, und eine Oberkörper-Lagerungsplatte 15, auf welcher der Oberkörper des Patienten 13 gelagert werden kann, auf.

Die Gantry 20 weist einen ersten Gantryteil 21, einen zweiten Gantryteil 22 und einen dritten Gantryteil 23 auf, wobei der erste Gantryteil 21 einen drehbar gelagerten Rotor 24 mit einem Projektionsdatenakquisitionssystem 27 aufweist, wobei der dritte Gantryteil 23 zumindest einen Abschnitt der Öffnung 9 aufweist, wobei die Haltestruktur 7 mittels der Schwenkvorrichtung 70 mit dem dritten Gantryteil 23 verbunden und relativ zu dem dritten Gantryteil 23 um die Schwenkachse 7A schwenkbar gelagert ist.

Der erste Gantryteil 21 ist relativ zu dem zweiten Gantryteil 22 und relativ zu dem dritten Gantryteil 23 derart bewegbar gelagert, dass eine Translationsbewegung des ersten Gantryteils 21 relativ zu dem zweiten Gantryteil 22 und dem dritten Gantryteil 23 ausgeführt werden kann, während gleichzeitig dazu der zweite Gantryteil 22 und der dritte Gantryteil 23 relativ zu dem Untersuchungsobjekt 14 ruhen und die Strahlenschutzvorrichtung 75 relativ zu dem Untersuchungsobjekt 14 und relativ zu dem zumindest einen Abschnitt der Öffnung 9 ruht, wenn sich das Untersuchungsobjekt 14 in der Öffnung 9 befindet.

Der erste Gantryteil 21 weist eine Drehlagerung 25 und eine Tragstruktur 26 auf, wobei der Rotor 24 mittels der Drehlagerung 25 mit der Tragstruktur 26 verbunden und relativ zu der Tragstruktur 26 um die Systemachse SA drehbar gelagert ist. Das Projektionsdatenakquisitionssystem 27 weist die Röntgenquelle 4A zum Erzeugen der Röntgenstrahlung 4 und den Röntgendetektor 4B zum Detektieren der Röntgenstrahlung 4 auf.

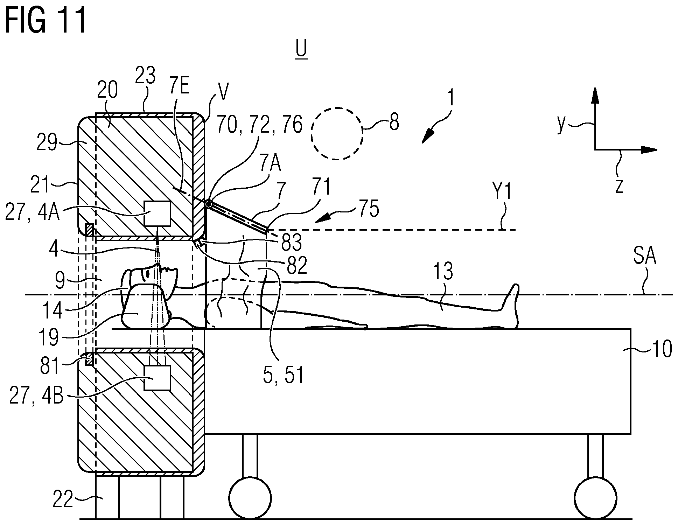

Die Fig. 11 zeigt ein Computertomographiegerät mit einer Gantry und einer Strahlenschutzvorrichtung zur Abdeckung einer Öffnung in einer weiteren Ansicht, wobei sich die Haltestruktur 7 in der zweiten Position befindet.

Das Computertomographiegerät 1 weist das Patientenbett 10 zur Lagerung des Patienten 13 auf. Die Gantry 20 weist einen Innenbereich 29 und eine Verkleidung V zum Abgrenzen des Innenbereichs 29 von einer Umgebung U auf, wobei die Haltestruktur 7 an einem Bereich der Verkleidung V anliegt, wenn sich die Haltestruktur 7 in der ersten Position der Haltestruktur 7 befindet, wobei die Haltestruktur 7 von dem Bereich der Verkleidung V wegragt, wenn sich die Haltestruktur 7 in der zweiten Position der Haltestruktur 7 befindet.

Die Haltestruktur 7 erstreckt sich flächig in einer Deckelebene 7E, wobei die Deckelebene 7E zu der Schwenkachse 7A im Wesentlichen parallel ist. Die Systemachse SA ist horizontal und parallel zu der horizontalen Richtung z. Die Schwenkachse 7A ist horizontal und senkrecht zu der horizontalen Richtung z. Die Schwenkachse 7A befindet sich oberhalb der Öffnung 9.

Die vertikale Position Y1 des ersten Rands 71 der Haltestruktur 7 in der zweiten Position der Haltestruktur 7 ist tiefer als in der ersten Position der Haltestruktur 7. In Bezug auf die vertikale Richtung y befindet sich ein Zwischenraum zwischen dem Patienten 13 und der Haltestruktur 7. Dadurch ist die Gefahr, dass der Patient 13 mit der Haltestruktur 7 kollidiert, verringert.

Das Computertomographiegerät 1 weist ferner ein Beleuchtungssystem 81 auf, welches dazu eingerichtet ist, die Öffnung 9 zumindest dann zu beleuchten, wenn sich die Haltestruktur 7 in der zweiten Position der Haltestruktur 7 befindet.

Das Computertomographiegerät 1 weist ferner ein Kamerasystem 82 auf, welches dazu eingerichtet ist, das Untersuchungsobjekt 14 zumindest dann optisch zu erfassen, wenn sich das Untersuchungsobjekt 14 in der Öffnung 9 befindet und wenn sich die Haltestruktur 7 in der zweiten Position der Haltestruktur 7 befindet. Das Kamerasystem 82 weist eine an der Haltestruktur 7 befestigte Kamera auf. Das Computertomographiegerät 1 weist den Bildschirm 38 auf. Ein von dem Kamerasystem 82 erzeugtes Bild des Untersuchungsobjekts 14 kann beispielsweise auf einem Bildschirm angezeigt werden.

Das Computertomographiegerät 1 weist ferner ein akustisches System 83 auf, welches dazu eingerichtet ist, ein akustisches Signal an das Untersuchungsobjekt 14 zumindest dann zu senden und/oder ein von dem Untersuchungsobjekt 14 ausgehendes akustisches Signal zumindest dann zu empfangen, wenn sich das Untersuchungsobjekt 14 in der Öffnung 9 befindet und wenn sich die Haltestruktur 7 in der zweiten Position der Haltestruktur 7 befindet. Das akustische System 83 weist ein Mikrofon und einen Lautsprecher auf, die an der Haltestruktur 7 befestigt sind.

## Patentansprüche

1. Computertomographiegerät (1), aufweisend eine Gantry (20) mit einer Öffnung (9) und eine Strahlenschutzvorrichtung (75) zur Abdeckung der Öffnung (9),
- wobei die Strahlenschutzvorrichtung (75) eine Haltestruktur (7), einen Strahlenschutzvorhang (5) und eine Schwenkvorrichtung (70) aufweist,
- wobei ein Untersuchungsobjekt (14) entlang einer Systemachse (SA) der Gantry (20) in die Öffnung (9) einführbar ist, wenn sich die Haltestruktur (7) in einer ersten Position relativ zu der Gantry (20) befindet,
- wobei die Haltestruktur (7) mittels der Schwenkvorrichtung (70) derart relativ zu der Gantry (20) um eine Schwenkachse (7A) schwenkbar gelagert ist, dass durch eine erste Schwenkbewegung der Haltestruktur (7) um die Schwenkachse (7A) die Haltestruktur (7) relativ zu der Gantry (20) von der ersten Position bis zu einer zweiten Position abgesenkt werden kann,
- wobei der Strahlenschutzvorhang (5) an der Haltestruktur (7) angeordnet ist,
- wobei ein unterer Bereich (5L) des Strahlenschutzvorhangs (5) von der Haltestruktur (7) herabhängt,
- **dadurch gekennzeichnet, dass** ein oberer Bereich (5H) des Strahlenschutzvorhangs (5) auf der Haltestruktur (7) aufliegt.

2. Computertomographiegerät (1) nach Anspruch 1,
- wobei der obere Bereich (5H) des Strahlenschutzvorhangs (5) auf der Haltestruktur (7) derart aufliegt, dass der obere Bereich (5H) des Strahlenschutzvorhangs (5) einen Bereich (8) einer Umgebung (U) der Gantry (20) vor Streustrahlen einer Röntgenstrahlung (4), die aus der Öffnung (9) kommend auf den Bereich (8) der Umgebung (U) der Gantry (20) gerichtet sind und die Haltestruktur (7) durchdringen, abschirmt.

3. Computertomographiegerät (1) nach Anspruch 1 oder 2,
- wobei der untere Bereich (5L) des Strahlenschutzvorhangs (5) und der obere Bereich (5H) des Strahlenschutzvorhangs (5) entlang einer Kante (5K) des Strahlenschutzvorhangs (5) aneinandergrenzen,
- wobei der Strahlenschutzvorhang (5) derart an der Haltestruktur (7) angeordnet ist, dass die Kante (5K) des Strahlenschutzvorhangs (5) entlang eines ersten Rands (71) der Haltestruktur (7) verläuft.

4. Computertomographiegerät (1) nach Anspruch 3,
- wobei der untere Bereich (5L) des Strahlenschutzvorhangs (5) und der obere Bereich (5H) des Strahlenschutzvorhangs (5) an der Kante (5K) des Strahlenschutzvorhangs (5) miteinander vernäht sind.

5. Computertomographiegerät (1) nach einem der Ansprüche 1 bis 4,
- wobei sich die Haltestruktur (7) flächig erstreckt,
- wobei der obere Bereich (5H) des Strahlenschutzvorhangs (5) auf der Haltestruktur (7) flächig aufliegt.

6. Computertomographiegerät (1) nach einem der Ansprüche 1 bis 5,
- wobei die Haltestruktur (7) aus Kunststoff hergestellt ist.

7. Computertomographiegerät (1) nach einem der Ansprüche 1 bis 6,
- wobei der Strahlenschutzvorhang (5) mittels eines Satzes von Druckknopf-Verbindungen (D) an der Haltestruktur (7) befestigt ist.

8. Computertomographiegerät (1) nach Anspruch 7,
- wobei der Strahlenschutzvorhang (5) einen Satz von vorhangseitigen Verbindungselementen (D5) aufweist,
- wobei die Haltestruktur (7) einen Satz von haltestrukturseitigen Verbindungselementen (D7) aufweist,
- wobei der Satz von vorhangseitigen Verbindungselementen (D5) und der Satz von haltestrukturseitigen Verbindungselementen (D7) zusammen den Satz von Druckknopf-Verbindungen (D) bilden.

9. Computertomographiegerät (1) nach Anspruch 8,
- wobei der Satz von haltestrukturseitigen Verbindungselementen (D7) eine erste Reihe von haltestrukturseitigen Verbindungselementen (D7) aufweist, wobei die erste Reihe von haltestrukturseitigen Verbindungselementen (D7) einem Verlauf des ersten Rands (71) der Haltestruktur (7) folgend angeordnet ist,
- wobei der Satz von vorhangseitigen Verbindungselementen (D5) eine erste Reihe von vorhangseitigen Verbindungselementen (D5) aufweist, wobei die erste Reihe von vorhangseitigen Verbindungselementen (D5) einem Verlauf der Kante (5K) des Strahlenschutzvorhangs (5) folgend und zu der ersten Reihe von haltestrukturseitigen Verbindungselementen (D7) korrespondierend angeordnet ist.

10. Computertomographiegerät (1) nach einem der Ansprüche 1 bis 9,
- wobei die Schwenkvorrichtung (70) zumindest ein Friktionsscharnier (76) aufweist,
- wobei die Haltestruktur (7) mittels des zumindest einen Friktionsscharniers (76) relativ zu der Gantry (20) um die Schwenkachse (7A) schwenkbar gelagert ist,
- wobei das zumindest eine Friktionsscharnier (76) dazu eingerichtet ist, die Haltestruktur (7) relativ zu der Gantry (20) in der zweiten Position zu halten.

11. Computertomographiegerät (1) nach einem der Ansprüche 1 bis 10,
- wobei die Schwenkvorrichtung (70) einen gantryseitigen Bereich (B2) aufweist,
- wobei die Haltestruktur (7) mittels der Schwenkvorrichtung (70) relativ zu dem gantryseitigen Bereich (B2) um die Schwenkachse (7A) schwenkbar gelagert ist,
- wobei die Gantry (20) einen Satz von Bolzen (C2) aufweist,
- wobei der gantryseitige Bereich (B2) einen Satz von Vertiefungen (C7) zur Aufnahme des Satzes von Bolzen (C2) aufweist,
- wobei der Satz von Bolzen (C2) derart in den Satz von Vertiefungen (C7) aufgenommen ist, dass der gantryseitige Bereich (B2) formschlüssig gegen eine Verschiebung relativ zu der Gantry (20) in einer Ebene, die zu der Systemachse (SA) und/oder zu den Bolzen des Satzes von Bolzen (C2) im Wesentlichen senkrecht ist, gesichert ist.

12. Computertomographiegerät (1) nach Anspruch 11,
- wobei die Bolzen des Satzes von Bolzen (C2) im Wesentlichen parallel zu der Systemachse (SA) ausgerichtet sind.

13. Computertomographiegerät (1) nach Anspruch 11 oder 12,
- wobei zumindest ein Bolzen des Satzes von Bolzen (C2) eine Nut (C21) aufweist,
- wobei der gantryseitige Bereich (B2) in zumindest einer Vertiefung des Satzes von Vertiefungen (C7) ein Sicherungselement (C71) aufweist,
- wobei das Sicherungselement (C71) senkrecht zu einer Axialrichtung des zumindest einen Bolzens derart in die Nut (C21) eingeführt ist, dass der gantryseitige Bereich (B2) formschlüssig gegen eine Verschiebung relativ zu der Gantry (20) in der Axialrichtung des zumindest einen Bolzens gesichert ist.

14. Computertomographiegerät (1) nach Anspruch 13,
- wobei der gantryseitige Bereich (B2) eine Feder (C0) aufweist, welche dazu eingerichtet ist, das Sicherungselement (C71) senkrecht zu der Axialrichtung des zumindest einen Bolzens gegen die Nut (C21) zu drücken.

15. Computertomographiegerät (1) nach Anspruch 13 oder 14,
- wobei der gantryseitige Bereich (B2) ein Bedienelement (C01) aufweist, welches an einer Oberfläche des gantryseitigen Bereichs (B2) angeordnet ist und mit dem Sicherungselement (C71) derart verbunden ist, dass durch einen Druck auf das Bedienelement (C01) ein Herausdrücken des Sicherungselements (C71) aus der Nut (C21) bewirkt werden kann.
